# EUROPEAN PATENT APPLICATION

(11) **EP 2 123 263 A1**
(43) Date of publication of application: **25.11.2009**
(21) Application number: 07714141.4
(22) Date of filing: 14.02.2007
(51) Int. Cl.: A61K 31/047, A61P 25/28

(54) **COMPOSITION AND METHOD FOR SUPPRESSING LIPID PEROXIDATION IN ERYTHROCYTES**

(71) Applicant: Tohoku University, Aoba-ku Sendai-shi Miyagi 980-8577 (JP); PROJECT M CO., LTD., Sendai-shi, Miyagi 980-8579 (JP)
(72) Inventor: MIYAZAWA, Teruo, Sendai-shi Miyagi 9808577 (JP); NAKAGAWA, Kiyotaka, Sendai-shi Miyagi 980-8577 (JP); KARIYA, Jun, Sendai-shi Miyagi 980-8579 (JP)
(74) Representative: Schwahn, Hartmut
(86) International application number: PCT/JP2007/052588
(87) International publication number: WO 2008/099469

(57) **Abstract**

The present invention relates to a composition for suppressing lipoperoxidation of erythrocytes, which comprises xanthophyll or an ester thereof as an active ingredient, a composition for preventing or treating dementia, and a method for diagnosing dementia, which comprises measuring the concentration of xanthophyll or an ester thereof in mammalian erythrocytes.

## Description

### Technical Field

The present invention relates to a composition and a method for suppressing lipoperoxidation of erythrocytes, a composition and a method for preventing or treating dementia, and a method for diagnosing dementia.

### Background Art

In Japan, the number of patients with dementia (elderly persons with dementia, having a degree of independence of II or higher) who needed nursing care and/or support was 1,490,000 (age of 65 years or greater; percentage of the population: 6.3%) as of 2002. The figure is estimated to be 3,780,000 (percentage in the population: 10.4%) in 2045. About one out of two persons certified as needing nursing care (need support) in 2002 was a patient with dementia. Around the world, the number of patients with dementia aged 60 or greater was 24,300,000 in 2001, and this number is estimated to increase to 81,000,000 in 2040. The number of patients with dementia is predicted to steadily increase as described above. Dementia is now a grave social issue. Alzheimer's disease accounts for the highest percentage among the causes of dementia, and one drug for delaying the progress of the symptoms is being marketed. Currently, there is no fundamental cure for the disease. Hence, development of an active ingredient for suppressing the onset or the progress of Alzheimer's disease is expected.

The present inventors have developed a chemiluminescence detection-HPLC method by which lipid hydroperoxide-that is, a primary oxidation product of biomembrane lipids-can be quantitatively determined selectively with high sensitivity. Patients with dementia including Alzheimer's disease patients, were subjected to blood analysis with the use of this method, so that abnormal accumulation of peroxidized phospholipids in erythrocyte membranes (2.8 times the average level for normal subjects) was discovered (Miyazawa T. Membrane phospholipid hydroperoxides as estimated by chemiluminescence: the effect of dietary polyunsaturated fatty acids. In: Sinclair A, Gibon R. ed. Essential Fatty Acids and Eicosanoids. Am. Oil Chem. Soc. Champaign, IL, (1993) 383-388; and Miyazawa T, Suzuki T, Yasuda K, Fujimoto K, Meguro K, Sasaki H. Accumulation of phospholipid hydroperoxides in red blood cell membranes in Alzheimer disease. In: Yagi K, Kondo M, Niki E, Yoshikawa T. ed. Oxygen Radicals. Elsevier, Amsterdam, (1992) 327-330). Therefore, it can be said that in patients with forms of dementia such as Alzheimer's disease many peroxidized erythrocytes (senescent erythrocytes) circulate *in vivo.*

Regarding erythrocytes of Alzheimer's disease patients, hemorheology alterations, such as decreased deformability and increased aggregation, have been reported. It has been reported that lipoperoxidation of erythrocytes is involved in aggravation of hemorheology (stiffening of erythrocytes or hemagglutination) and rats with hemorheology alterations exhibited lowered cognitive functions. Based on research concerning β-amyloid in recent years, it has now been considered that such abnormalities in erythrocytes are due to exposure of β-amyloid in blood to erythrocytes. Lipoperoxidation (aging) of erythrocytes via β-amyloid causes hemorheology alterations, resulting in reduction in brain blood flow and reduced oxygen transport to the brain. This has also been thought to be one pathway involved in the onset or aggravation of Alzheimer's disease. The present inventors have searched for ingredients capable of preventing lipoperoxidation of erythrocytes, but they have revealed that α-tocopherol, which is known well as an antioxidant, has no such effects.

### Disclosure of the Invention

An object of the present invention is to provide a means for preventing lipoperoxidation of erythrocytes and preventing or treating dementia, and a method for diagnosing dementia.

As a result of studies to attain the above object, the present inventors have discovered the following. The concentration of xanthophyll in erythrocytes is negatively correlated with the peroxidized phospholipid level in erythrocytes. Xanthophyll tends to be transferred to erythrocytes, and accumulation of peroxidized phospholipids can be suppressed by increasing the concentration of xanthophyll in erythrocytes. Thus, the present inventors have completed that the present invention.

The present invention encompasses the following (1) to (13).
(1) A composition for suppressing lipoperoxidation of erythrocytes, comprising xanthophyll or an ester thereof as an active ingredient.
(2) A composition for preventing or treating dementia, comprising xanthophyll or an ester thereof as an active ingredient.
(3) The composition according to (1) or (2), wherein the xanthophyll is lutein.
(4) The composition according to (2), wherein the dementia is Alzheimer's disease.
(5) The composition according to any one of (1) to (4), which is a food composition.
(6) A method for diagnosing dementia, comprising measuring the concentration of xanthophyll or an ester thereof in mammalian erythrocytes.
(7) The diagnostic method according to (6), wherein the xanthophyll is lutein.
(8) The diagnostic method according to (6) or (7), wherein the dementia is Alzheimer's disease.
(9) A method for suppressing lipoperoxidation of erythrocytes, comprising administering an effective amount of xanthophyll or an ester thereof to a mammal.
(10) A method for preventing or treating dementia, comprising administering an effective amount of xanthophyll or an ester thereof to a mammal.
(11) Use of xanthophyll or an ester thereof for preparation of a composition for suppressing lipoperoxidation of erythrocytes.
(12) Use of xanthophyll or an ester thereof for preparation of a composition for preventing or treating dementia.
(13) The use according to (11) or (12), wherein the xanthophyll is lutein.

### Brief Description of the Drawings

Fig. 1 is an example of an UV-HPLC chart showing the concentrations of erythrocyte carotenoid in Alzheimer's disease patients and normal subjects as measured by UV-HPLC.
Fig. 2 shows the results of measuring the concentrations of erythrocyte carotenoid in Alzheimer's disease patients and normal subjects.
Fig. 3 shows an example of a CL-HPLC chart showing the peroxidized phospholipid levels in erythrocytes as measured by CL-HPLC.
Fig. 4 shows the results of administering lutein to 6 normal subjects for 4 weeks and collecting blood from the normal subjects after they had fasted for 12 hours at the following time points: before ingestion; 2 weeks after ingestion; and 4 weeks after ingestion. This was followed by measurement of the concentration of erythrocyte xanthophyll and the peroxidized phospholipid level.

### Embodiments of the Invention

The present inventors have discovered that the concentration of xanthophyll in erythrocytes is negatively correlated with the peroxidized phospholipid level, and accumulation of peroxidized phospholipids can be suppressed by increasing the concentration of xanthophyll in erythrocytes. The present invention is based on the above findings. Therefore, in an embodiment, the present invention relates to a composition for suppressing lipoperoxidation of erythrocytes, which comprises xanthophyll as an active ingredient.

The term "xanthophyll" in this description refers to a group of carotenoid having oxygen in the form of hydroxy group, carbonyl group, epoxy group, methoxy group, or the like. Examples of xanthophyll include lutein, zeaxanthin, cryptoxanthin, fucoxanthin, violaxanthin, neoxanthin, astaxanthin, and canthaxanthin. They may be used independently or two or more types thereof may be used in combination. In the present invention, xanthophyll is preferably lutein. An ester of xanthophyll is a mono ester or diester with long-chain fatty acid, and preferably, a mono ester or diester with C₁₂₋₁₈ fatty acid such as lauric acid, myristic acid, palmitin acid, and stearic acid. As xanthophyll, synthetic xanthophyll may be used or an extract from a natural product (e.g., chlorella or green and yellow vegetables) may also be used.

The expression "suppressing lipoperoxidation of erythrocytes" refers to suppression of peroxidation of lipids existing in erythrocytes, particularly phospholipids, and more specifically phospholipids composing erythrocyte membranes. The erythrocyte membrane is composed of a lipid bilayer, in which the major constituents are phosphatidylcholine (PC) and phosphatidylethanolamine (PE). Therefore, in this description, the expression "lipoperoxidation of erythrocytes" refers to preferably peroxidation of phosphatidylcholine (PC) and phosphatidylethanolamine (PE) composing the erythrocyte membrane. When these phospholipids composing the erythrocyte membrane are peroxidized, phosphatidyl choline hydroperoxide (PCOOH) is generated from PC and phosphatidylethanolamine hydroperoxide (PEOOH) is generated from PE. All of them have hydroperoxide-type unsaturated fatty acid at the sn2 position of the glycerol backbone of phospholipids. Therefore, lipoperoxidation of erythrocytes can be detected by chemiluminescence detection-high performance liquid chromatography (CL-HPLC), by which PCOOH and PEOOH can be quantitatively determined specifically with ultra-high-sensitivity (T. Miyazawa, T. Suzuki, K. Fujimoto, K. Yasuda, J. Lipid Res., 33, 1051-1059 (1992); T. Miyazawa, K. Fujimoto, T. Suzuki, K. Yasuda, Methods Enzymol., 233, 324-332 (1994)). This method involves HPLC fractionation by lipid class, reacting a hydroperoxide (-OOH) group in each lipid class with a luminescence reagent comprising a mixed solution of cytochrome C and luminol, and then detecting with a post-column chemiluminescence detector.

Lipoperoxidation (aging) of erythrocytes causes hemorheology alterations, resulting in reduction in brain blood flow and reduction in oxygen transport to the brain. It has also been reported that this is involved in the onset or aggravation of dementia. Also, such erythrocytes are in a state such that oxygen is distributed richly in the phospholipid membrane. Furthermore, oxygen dissociation from oxidized hemoglobin is inhibited, resulting in chronic oxygen deficiency in brain tissues. This is also thought to be involved in the onset or aggravation of dementia (S. B. Solerte, G. Ceresini, E. Ferrari, M. Fioravanti, Neurobiol. Aging, 21, 271-281 (2000); R.S. Ajmani, E. J. Metter, R. Jaykumar, D. K. Ingram, E.L. Spangler, O.O. Abugo, J. M. Rifkind, Neurobiol. Aging, 21 257-269 (2000)). Therefore, in one embodiment, the present invention relates to a composition for preventing or treating dementia, which comprises xanthophyll as an active ingredient.

In the description, the term "dementia" is used in connection with conditions that are not associated with perception disorder or consciousness disorders due to various diseases including serious infections and toxins, but are generally associated with brain tissue diseases. Furthermore, the term "dementia" can be characterized by progressive loss of cognitive functions and intellectual functions and specifically characterized by disorientation, memory disorder, disorder concerning decision, dysmentia, and slight affective changes. Examples of dementia include, but are not limited to, Alzheimer's disease, AIDS dementia, presenile dementia, senile dementia, catatonic dementia, dialysis dementia (dialysis encephalopathy syndrome), epileptic dementia, hebephrenic dementia, Lewy body dementia (diffuse Lewy body disease), multiinfarct dementia (vascular dementia), dementia paralytica, posttraumatic dementia, precocious dementia, primary dementia, toxic dementia, and vascular dementia. The composition for preventing or treating dementia of the present invention is particularly appropriate for preventing or treating Alzheimer's disease.

In this description, the term "prevention of (preventing) dementia" refers to suppressing and delaying the onset of dementia. The term "treatment of (or "treating of") dementia" refers to the curing of dementia, the improvement of symptoms, and the suppression of the progress of the symptoms.

Hereinafter, both a composition for suppressing lipoperoxidation of erythrocytes, which comprises xanthophyll as an active ingredient, and a composition for preventing or treating dementia, which comprises the same, are referred to as the composition of the present invention.

Subjects of administration of the composition of the present invention are mammals. In the description, the term "mammal(s)" refers to warm blooded vertebrate(s). Examples of such vertebrates include primates such as humans and monkeys, rodents such as mice, rats, and rabbits, pet animals such as dogs and cats, and domestic animals such as cattle, horses, and pigs. Subjects for administration are preferably primates such as monkeys and chimpanzees and more preferably humans. The composition of the present invention is administered particularly preferably to humans with dementia, humans diagnosed to have dementia, humans who may be affected with dementia, and humans who need prevention of dementia.

The composition of the present invention is not particularly limited and can be prepared as a pharmaceutical composition or a food composition, for example.

When the composition of the present invention is prepared as a pharmaceutical composition, the composition is generally prepared as a preparation comprising xanthophyll and preferably a pharmaceutically acceptable carrier. The pharmaceutical composition is administered orally or parenterally, such as subcutaneously, intravenously, intraarterially, intramuscularly, intraperitoneally, or intranasally.

The term "pharmaceutically acceptable carrier" generally refers to inactive, atoxic, and solid or liquid extending agent, diluent, encapsulation material, or the like that is not reactive with the active ingredients of the present invention. Examples of such pharmaceutically acceptable carrier include water, ethanol, polyol (e.g., glycerol, propylene glycol, and liquid polyethylene glycol), appropriate mixtures thereof, and solvents such as vegetable oil or dispersion medium.

The pharmaceutical composition is preferably in the form of preparation for oral administration. Examples of such form include tablets, granules, subtle granules, powders, capsules, syrups, and solutions.

The pharmaceutical composition may further contain an additive conventionally used in medical fields. Examples of such additive include excipients, binders, disintegrators, lubricants, antioxidants, colorants, and flavoring agents, which can be used if necessary. To enable sustained release of the pharmaceutical composition, so as to cause the composition to act over a long time period, the composition can also be coated with a known retarder or the like. Examples of an excipient that can be used herein include sodium carboxymethylcellulose, agar, light anhydrous silicic acid, gelatin, crystalline cellulose, sorbitol, talc, dextrin, starch, lactose, saccharose, dextrose, mannitol, magnesium metasilicate aluminate, and calcium hydrogen phosphate. Examples of a binder include gum Arabic, sodium alginate, ethanol, ethyl cellulose, casein sodium, sodium carboxymethylcellulose, agar, purified water, gelatin, starch, tragacanth, lactose, hydroxycellulose, hydroxymethylcellulose, hydroxypropylcellulose, and polyvinylpyrrolidone. Examples of a disintegrator include carboxymethylcellulose, sodium carboxymethylcellulose, carboxymethylcellulose calcium, crystalline cellulose, starch, and hydroxypropyl starch. Examples of a lubricant include stearic acid, calcium stearate, magnesium stearate, talc, hardened oil, sucrose fatty acid ester, and waxes. Examples of an antioxidant include tocopherol, gallic acid ester, dibutylhydroxytoluene (BHT), butyl hydroxyanisole (BHA), and ascorbic acid. Another additive or remedy such as an antacid (e.g., sodium hydrogencarbonate, magnesium carbonate, precipitated calcium carbonate, and synthetic hydrotalcite) or a gastric coating agent (e.g., synthetic aluminum silicate, sucralfate, and sodium copper chlorophyllin) may also be added, as needed.

The pharmaceutical composition may also be administered parenterally in the form of sustained-release subcutaneous implant or standard delivery system (e.g., monoclonal antibody, vector delivery, ion implantation, polymer matrix, liposome, and microsphere).

When the composition of the present invention is prepared as a food composition, the form thereof is not particularly limited and includes beverages, health foods, and functional foods, for example. Health foods and functional foods may exist in the form of various preparations. Specific examples of such preparations include subtle granules, tablets, granules, powders, capsules, syrups, solutions, and fluid diets. A food composition in the form of a preparation can be produced using conventional means after addition of an appropriate excipient (e.g., starch, processed starch, lactose, dextrose, and water). Furthermore, the food composition may be in the form of a liquid food composition such as a soup, juice, milk beverage, cocoa, and jelly-like beverage, a semisolid food composition such as pudding and yogurt, bread, sweets, noodles such as wheat noodles, sweets such as cookies, chocolate, candies, and Japanese crackers, and a topping (spread) such as *Furikake* (rice topping), butter, and jam.

The food composition may be mixed with various food additives such as antioxidants, flavoring agents, various esters, organic acids, organic acid salts, inorganic acids, inorganic acid salts, inorganic salts, dyes, emulsifiers, preservatives, seasonings, sweeteners, acidulants, fruit juice extracts, vegetable extracts, nectar extracts, pH adjusting agents, and quality stabilizers independently or in combination.

The dose of the composition of the present invention can be appropriately determined depending on the route of administration and the patient's age, symptoms, and the like. The dose in terms of the mass of xanthophyll generally ranges from 0.01 mg to 2 mg/kg and preferably ranges from 0.1 mg to 0.2 mg/kg body weight per day via oral administration.

The amount of xanthophyll in the composition of the present invention can be appropriately determined to achieve the above dose. When the composition is in the form of tablets, granules, subtle granules, powders, capsules, or the like, the amount of xanthophyll in the composition generally ranges from 0.35% by mass to 70 % by mass, and preferably ranges from 3.5% by mass to 7% by mass.

To the composition of the present invention, for example, a functional component such as vitamin C, vitamin E, vitamin B12, vitamin B2, vitamin B1, β-carotene, astaxanthin, tocotrienol, minerals (e.g., calcium, magnesium, iron, and zinc), phosphatidylcholine, phosphatidylserine, plasmalogen, a *ginkgo biloba* extract, wine polyphenol, green tea catechin, theaflavin, perilla oil, DHA (docosahexaenoic acid), and chlorella can be appropriately added.

Through administration of an effective amount of the composition of the present invention to a mammal (that is, administration of an effective amount of xanthophyll to a mammal), lipoperoxidation of erythrocytes can be suppressed and thus dementia can be prevented or treated.

Conventional dementia drugs are intended for treatment and adverse effects thereof are concerned. Meanwhile, xanthophyll is a safe food ingredient that we have long experience of eating. Xanthophyll is advantageous in that it can prevent the onset of dementia and slow down the progress of symptoms through nutrition guidance and the like in general eating habits.

The present inventors have discovered that the concentration of xanthophyll in erythrocytes is negatively correlated with the peroxidized phospholipid level. They further discovered that the concentration of xanthophyll in erythrocytes of patients with dementia is significantly lower than that of normal subjects. Therefore, in an embodiment, the present invention relates to a method for diagnosing dementia, and preferably Alzheimer's disease, comprising measuring the concentration of xanthophyll, and preferably the concentration of lutein, in mammalian erythrocytes. Here, the concentration of xanthophyll in erythrocytes can be measured as the number of moles (mol/g Hb) of xanthophyll per gram of hemoglobin, for example. The diagnosis of dementia in the present invention includes diagnosis of affections associated with dementia and the possibility of having dementia.

In one embodiment, the diagnostic method of the present invention comprises the steps of separating erythrocytes from mammalian blood, extracting xanthophyll from erythrocytes, and measuring xanthophyll. Erythrocytes can be separated from blood by centrifugation of collected blood, for example. Washing is preferably performed by repeating a step of adding a physiological phosphate buffer or the like to the separated erythrocytes and then performing centrifugation to remove supernatants. Erythrocytes are preferably subjected to hemolysis before extraction of xanthophyll.

Examples of an extraction solvent to be used for extraction of xanthophyll from erythrocytes is not particularly limited and include hexane, heptane, benzene, dichloromethane, chloroform, acetone, acetonitrile, cyclohexane, toluene, and esters such as ethyl acetate, ethers such as 1,4-dioxane and tetrahydrofuran, and C₁₋₆ lower alcohol such as methanol, ethanol, and isopropanol. As an extraction solvent, a mixture of hexane and dichloromethane (hexane/dichloromethane, preferably, hexane : dichloromethane = 5:1 (v/v)) is preferably used. To an extraction solvent, further pyrogallol (ethanol solution) and a potassium hydroxide aqueous solution are added preferably. Degradation of erythrocyte xanthophyll during an extraction process can be prevented by the addition of pyrogallol. A potassium hydroxide aqueous solution degrades erythrocyte proteins and the like that are contaminants, thereby contributing to improvement of the extraction rate of xanthophyll from erythrocytes.

After extraction, the extraction solvent is removed, the residue is dissolved in hexane/acetone (mixture of hexane and acetone), and then impurities are preferably removed by chromatography, preferably by silica gel column chromatography.

More specifically, steps of extracting xanthophyll from erythrocytes are as follows:
a step of adding water to erythrocytes to hemolyze the erythrocytes;
a step of adding pyrogallol (ethanol solution), potassium hydroxide (KOH) aqueous solution, hexane/dichloromethane solution (preferably, 5:1 (v/v)), and dibutylhydroxytoluene and then mixing them,
a step of repeating collection of the upper layer (hexane/dichloromethane layer) and extraction using hexane/dichloromethane;
a step of joining the collected upper layers and then removing the solvent therefrom;
a step of dissolving the residue in hexane/acetone (preferably, 2:1 (v/v)) and then removing impurities by chromatography, preferably silica gel column chromatography; and
a step of running hexane/acetone (preferably, 2:1 (v/v)), so as to collect the eluent.

A method for measuring extracted xanthophyll is not particularly limited. Extracted xanthophyll can be measured by a conventional method such as methods using high performance liquid chromatography. Specifically, xanthophyll can be measured using UV-HPLC, multiple wavelength UV-HPLC, a high performance liquid chromatography mass spectroscope (LC-MS, LC-MS/MS), fluorescence detection-high performance liquid chromatography (FL-HPLC), light scattering detection-high performance liquid chromatography (HPLC-ELSD), electrochemical detection-high performance liquid chromatography, or the like. When UV-HPLC is used, examples of a column to be used herein include a C8 column, a C18 column, a C30 column, a silica column, and a phenyl column. For example, a C30 column (Carotenoid S-5 µm 4.6 × 250 mm) manufactured by YMC can be used. In addition, measurement conditions for UV-HPLC may be appropriately determined. For example, measurement can be carried out under the following conditions.

Column oven temperature: 10°C to 30°C, preferably, 15°C to 25°C

Pump flow rate: 0.5 mL/min to 2 mL/min, preferably, 0.8 mL/min to 1.5 mL/min

Mobile phase A: methanol/t-butyl methyl ether/water (containing ammonium acetate)

Mobile phase B: methanol/t-butyl methyl ether/water (containing ammonium acetate)

Detection wavelength: 463 nm

An advantage of the method for diagnosing dementia comprising measuring xanthophyll is to make it possible to predict early the onset of dementia conveniently and rapidly through a blood test upon health examination or the like.

### Examples

The present invention is hereafter described in greater detail with reference to the following examples.

### (Example 1) Measurement of the concentration of erythrocyte carotenoid in Alzheimer's disease patients and normal subjects

Alzheimer's disease patients (24 patients; 11 male patients and 13 female patients) and normal partners thereof (24 normal partners; 13 male partners and 11 female partners) were subjected to measurement of the concentration of erythrocyte carotenoid. The concentration of erythrocyte carotenoid was measured as follows.

### 1-1. Preparation of packed cells (washed erythrocytes) from erythrocytes

Blood (12 mL) of a subject after the subject had fasted for 12 hours was collected using EDTA. Blood was centrifuged (1000 g, 10 minutes, 4°C), so that 6 mL of erythrocytes was obtained. To wash erythrocytes, 5-fold volume (30 mL) of physiological phosphate buffer (pH 8.0) was added and then centrifugation was carried out (2300 g, 10 minutes, 4°C), thereby removing supernatants. This procedure was repeated 3 times, so that 4 mL of packed cells (washed erythrocytes) was obtained.

### 1-2. Extraction of carotenoid from erythrocytes and quantitative determination thereof

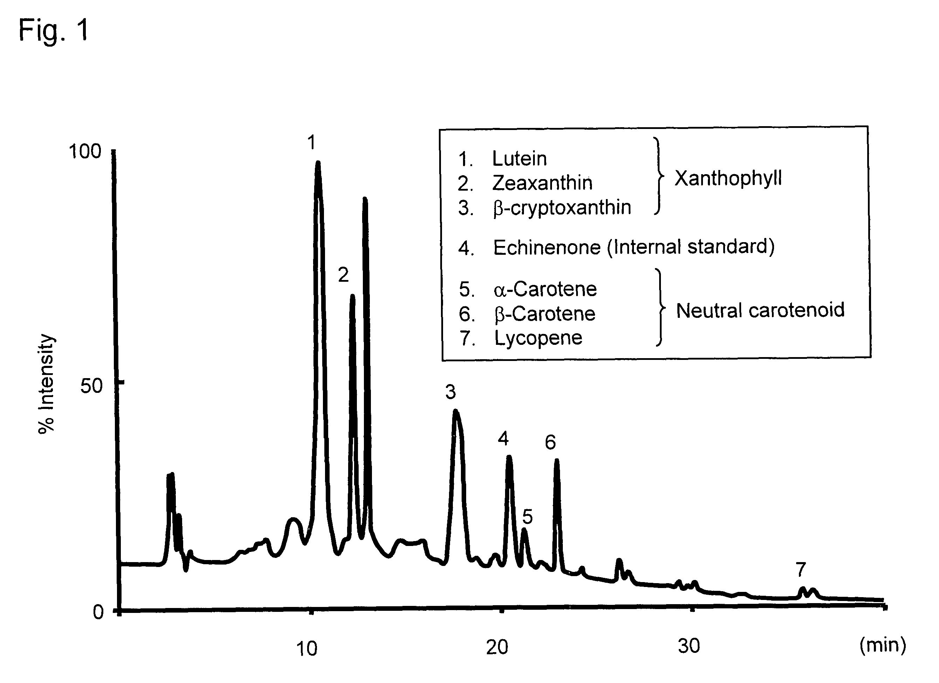

Two (2) mL of water was added to 2 mL of packed cells (washed erythrocytes) for hemolysis of erythrocytes. To the solution, 4 mL of 80 mM pyrogallol (ethanol solution), 50 µL of 1 µM internal standard echinenone (ethanol solution), 0.8 mL of a 1.8 M potassium hydroxide (KOH) aqueous solution, 1.0 mL of a 100 mM sodium dodecyl sulfate (SDS) aqueous solution, and 12 mL of a hexane/dichloromethane solution (5:1, v/v, 1.2 mM dibutylhydroxytoluene; containing BHT) were added. The mixture was then subjected to ultrasonication in ice for 5 minutes, vortexed for 3 minutes, and then centrifuged (1000 g, 10 minutes, 4°C). An upper layer (hexane/dichloromethane layer) was collected in a pear flask and then subjected to extraction with hexane/dichloromethane once again. The thus collected upper layers were joined and then subjected to solvent removal under nitrogen. The residue was dissolved in 3 mL of hexane/acetone (2:1, v/v) and then the solution was subjected to Sep-Pak (trademark) Plus Silica Cartridges (Waters), so as to remove impurities. Further 7 mL of hexane/acetone (2:1, v/v) was run, so that 10 mL of an eluent was recovered. The residue obtained via solvent removal under nitrogen was dissolved again in 100 µL of a methanol/t-butyl methyl ether solution (2:3, v/v). Fifty (50) µL of the solution was subjected to the following UV-HPLC analysis, so that erythrocyte carotenoid was quantitatively determined. In addition, to prevent isomerization or degradation of carotenoid due to light, all extraction procedures were carried out under an FL20S.Y-F yellow fluorescent lamp (Panasonic). Fig. 1 shows an example of the thus obtained UV-HPLC chart.

### UV-HPLC analysis conditions

Column: C30 Carotenoid (5 µm, 4.6 x 250 mm, YMC)
Column oven: 860-CO (JASCO) Temperature: 20°C
Pump: 880-PU (JASCO) Flow rate: 1 mL/min
Mobile phase A: methanol/t-butyl methyl ether/water = 83:15:2 (v/v/v, containing 3.9 mM ammonium acetate)
Mobile phase B: methanol/t-butyl methyl ether/water = 8:90:2 (v/v/v, containing 2.6 mM ammonium acetate)
UV detector: NANOSPACE SI-1 (SHISEIDO)
Detection wavelength: 463 nm

**Table 1**

| Gradient conditions | | | | | | | |
|---|---|---|---|---|---|---|---|
| Time | 0 | 5 | 17 | 29 | 45 | 47 | (minute) |
| A | 90 | 90 | 55 | 5 | 5 | 90 | (%) |
| B | 10 | 10 | 45 | 95 | 95 | 10 | (%) |

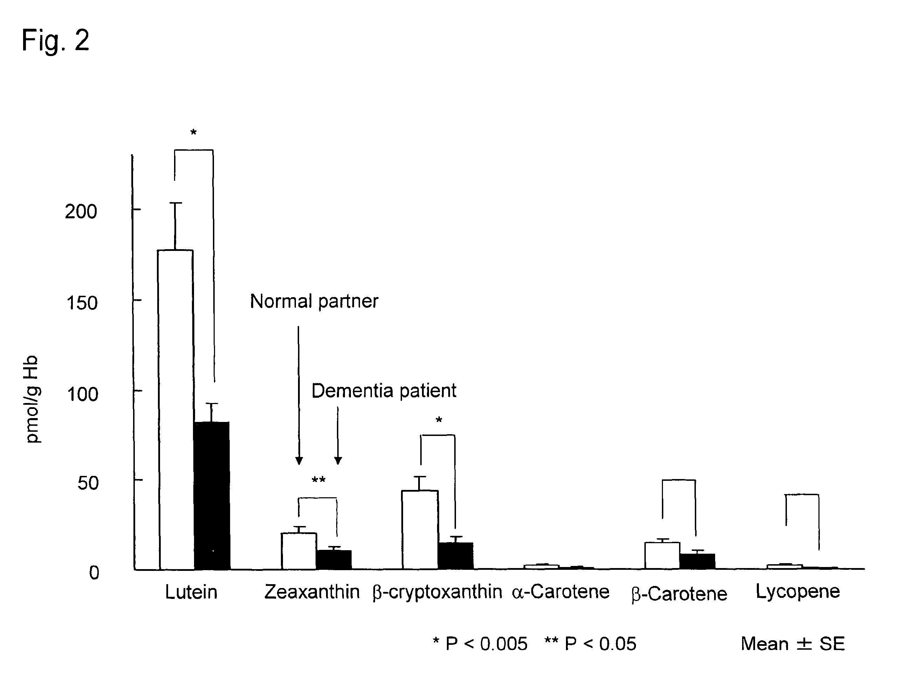

Fig. 2 shows the results of UV-HPLC measurement. As shown in Fig. 2, the erythrocyte xanthophyll concentration and particularly the erythrocyte lutein concentration in patients with dementia was significantly lower than that in normal subjects (P < 0.005). Also with regard to carotenoids (specifically, α-carotene, β-carotene, and lycopene) other than xanthophyll, differences were observed between patients with dementia and normal subjects, although the differences were not so significant as in the case of xanthophyll.

### (Example 2) Measurement of xanthophyll concentration and peroxidized phospholipid level in erythrocytes

Twenty (20) normal subjects were subjected to measurement of the total erythrocyte xanthophyll (lutein + zeaxanthin + β-cryptoxanthin) concentration and the peroxidized phospholipid level. The total erythrocyte xanthophyll concentration was measured by a method similar to the method used for measuring the concentration of erythrocyte carotenoid in Example 1. The peroxidized phospholipid level was measured as follows.

### 2-1. Extraction of lipids from erythrocytes

Packed cells (washed erythrocytes, 1.5 mL) prepared in a manner similar to that in 1-1 of Example 1 were added to a 50-mL screw cap glass centrifugation tube and then 1.5 mL of a 0.1 mM EDTA-2Na aqueous solution was added, followed by gentle agitation. The solution was allowed to stand in ice for 15 minutes and then 15 mL of isopropanol was added, followed by gentle agitation. The solution was allowed to stand for 30 minutes in ice and then 15 mL of chloroform was added. After vortexing, the solution was allowed to stand for 30 minutes in ice and then centrifuged (1000 g, 10 minutes, 4°C). One layer of the extract was collected in a pear flask and then the solvent was removed using an evaporator. The thus obtained residue was dissolved in 2.5 mL of chloroform/methanol (2 : 1, v/v) and then the resultant was transferred to a test tube. Water (0.5 mL) was added to the test tube, followed by vortexing and centrifugation (1000 g, 10 minutes, 4°C). The thus obtained lower layer was transferred to another test tube and then 1.8 mL of chloroform/methanol (10 : 1, v/v) was added to the remaining upper layer. Similarly, vortexing and centrifugation were carried out and then the thus obtained lower layer and the previously obtained lower layer were joined. Solvent removal was carried out under nitrogen and then the total lipid level was measured by a gravimetric method. Total erythrocyte lipids, the residue, were dissolved again in 100 µL of chloroform/methanol (2 : 1, v/v).

### 2-2. Measurement of erythrocyte peroxidized phospholipids

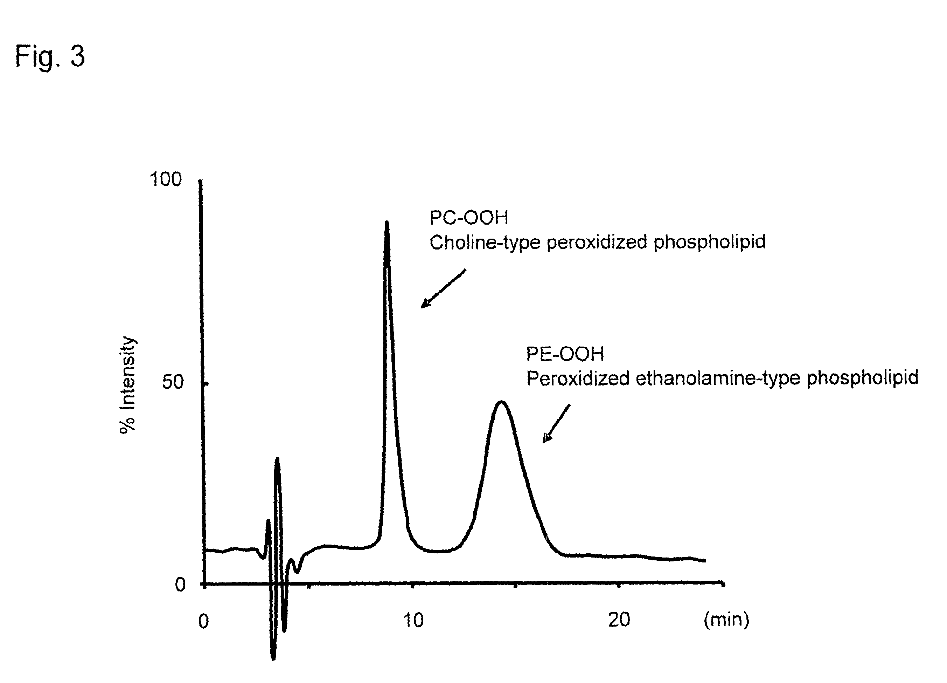

Peroxidized phospholipids (phosphatidyl choline hydroperoxide (PCOOH) and phosphatidylethanolamine hydroperoxide (PEOOH)) in erythrocytes were measured by chemiluminescence-high performance liquid chromatography (CL-HPLC). The CL-HPLC apparatus is a combination of high performance liquid chromatography (HPLC) and a chemiluminescence detector (CL). After separation of PCOOH and PEOOH from other lipid components by HPLC, PCOOH and PEOOH are reacted with a luminescence reagent and then hydroperoxide-group-derived chemiluminescence is detected and quantitatively determined. Specifically, the above obtained chloroform/methanol (2 : 1, v/v) solution (40 µL) of total erythrocyte lipids was subjected to CL-HPLC and then peroxidized phospholipids were quantitatively determined. Fig. 3 shows an example of the thus obtained CL-HPLC chart.
CL-HPLC analysis conditions
HPLC pump: 880-PU (JASCO)
HPLC column: Finepak SIL-NH₂, 4.6 mmID × 250 mm (JASCO) Mobile phase: isopropylalcohol/methanol/water (130/45/25, v/v/v) Flow rate: 1.0 mL/min
Column oven: 860-CO (JASCO) Temperature: 40°C Luminescence reagent: Prepared by dissolving 2 µM luminol (Wako Pure Chemical Industries, Ltd.) and 25 µM cytochrome c (Sigma, type VI) in a 50 mM borate buffer (pH 10).
Flow rate: 1.8 ml/min
Chemiluminescence detector: CLD-100 (Tohoku Electronic Industrial Co., Ltd.)

As a result, the total erythrocyte xanthophyll concentration was negatively correlated with the peroxidized phospholipid level (r = -0.46, P = <0.05). This suggests that accumulation of peroxidized phospholipids can be suppressed by increasing the concentration of erythrocyte xanthophyll.

### Example 3 Administration of xanthophyll

To examine whether the ingested xanthophyll would be actually transferred to erythrocytes so as to suppress accumulation of peroxidized phospholipids, lutein that exists at the highest concentration among human erythrocyte xanthophyll members was administered to 6 normal subjects (3 male subjects and 3 female subjects) for 4 weeks. The subjects ingested one capsule containing lutein after breakfast every day for 4 weeks. This means ingestion of 9.7 mg of lutein/day (2.1 times the daily ingestion amount). Blood (12 mL each) was collected using EDTA from a normal subject that had fasted for 12 hours at the following time points: before ingestion; two weeks after ingestion; and four weeks after ingestion. Erythrocytes were separated from the thus obtained blood and then the concentration of erythrocyte lutein and the peroxidized phospholipid level were measured. The concentration of erythrocyte lutein and the peroxidized phospholipid level were measured in a manner similar to those used in Examples 1 and 2.

Subsequently, zeaxanthin was similarly tested in an ingestion amount (0.7 mg/day; approximately twice the daily ingestion amount).

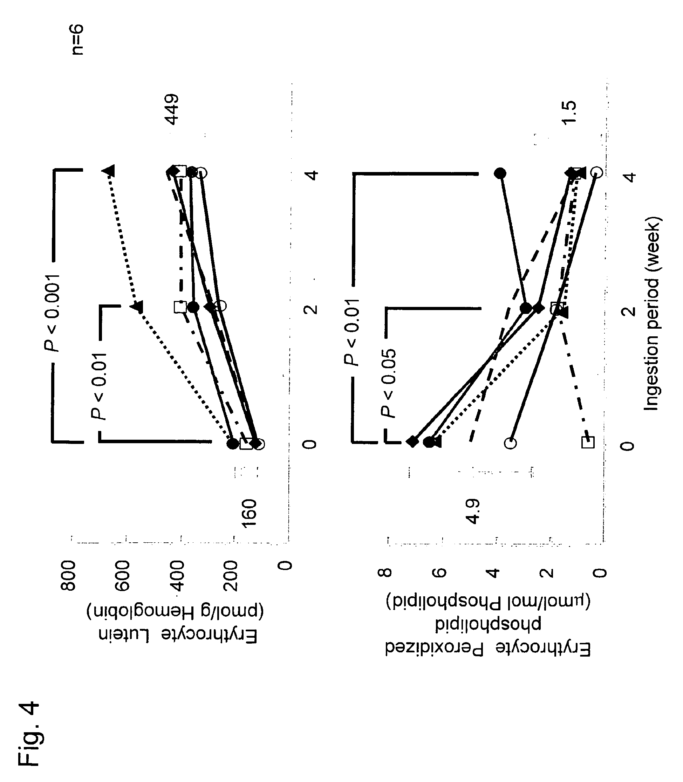

Before ingestion, the concentration of erythrocyte lutein was 160 pmol/g hemoglobin in average. On week 2 after the start of ingestion, the concentration was found to have significantly increased. The concentration reached 449 pmol/g hemoglobin (2.8 times the amount before the start of ingestion) after 4 weeks (Fig. 4). On the other hand, the peroxidized phospholipid level was found to have significantly decreased within 4 weeks (after the start of ingestion) compared with the level measured before the start of ingestion. It was demonstrated that the ingested lutein was aggressively transferred to and accumulated in erythrocytes, so as to suppress accumulation of peroxidized phospholipids.

The concentration of erythrocyte zeaxanthin measured before the start of ingestion was 52 pmol/g hemoglobin in average. After 4 weeks, the concentration of erythrocyte zeaxanthin reached 71 pmol/g hemoglobin, which was not significant (P = 0.08). Nevertheless, this concentration showed an increasing tendency.

## Claims

1. A composition for suppressing lipoperoxidation of erythrocytes, comprising xanthophyll or an ester thereof as an active ingredient.

2. A composition for preventing or treating dementia, comprising xanthophyll or an ester thereof as an active ingredient.

3. The composition according to claim 1 or 2, wherein the xanthophyll is lutein.

4. The composition according to claim 2, wherein the dementia is Alzheimer's disease.

5. The composition according to any one of claims 1 to 4, which is a food composition.

6. A method for diagnosing dementia, comprising measuring the concentration of xanthophyll or an ester thereof in mammalian erythrocytes.

7. The diagnostic method according to claim 6, wherein the xanthophyll is lutein.

8. The diagnostic method according to claim 6 or 7, wherein the dementia is Alzheimer's disease.

9. A method for suppressing lipoperoxidation of erythrocytes, comprising administering an effective amount of xanthophyll or an ester thereof to a mammal.

10. A method for preventing or treating dementia, comprising administering an effective amount of xanthophyll or an ester thereof to a mammal.

11. Use of xanthophyll or an ester thereof for preparation of a composition for suppressing lipoperoxidation of erythrocytes.

12. Use of xanthophyll or an ester thereof for preparation of a composition for preventing or treating dementia.

13. The use according to claim 11 or 12, wherein the xanthophyll is lutein.
